# EUROPÄISCHE PATENTSCHRIFT

(11) **EP 1 781 251 B1**
(45) Veröffentlichungstag und Bekanntmachung des Hinweises auf die Patenterteilung: **05.05.2010**
(21) Anmeldenummer: 05773352.9
(22) Anmeldetag: 25.08.2005
(51) Int. Cl.: A61K 9/00, A61L 27/16, A61L 27/52, A61F 2/44

(54) **POLYVINYLALKOHOL GELE, INSBESONDERE IN SITU GELIEREND**
POLYVINYL ALCOHOL GELS, ESPECIALLY IN SITU GELLING GELS
GELS D'ALCOOL POLYVINYLIQUE, NOTAMMENT GELS A GELIFICATION SITU

(30) Priorität: 25.08.2004 DE 102004041368; 14.10.2004 DE 102004050254
(43) Veröffentlichungstag der Anmeldung: 09.05.2007
(73) Patentinhaber: InnoGEL AG, 6331 Hünenberg (CH)
(72) Erfinder: MÜLLER, Rolf, CH-8055 Zürich (CH); INNEREBNER, Federico, CH-8049 Zürich (CH)
(74) Vertreter: Becker Kurig Straus
(86) Internationale Anmeldenummer: PCT/CH2005/000497
(87) Internationale Veröffentlichungsnummer: WO 2006/021122

(56) Entgegenhaltungen:
- WO-A-2004/098756
- US-A1- 2001 029 399
- US-A1- 2002 034 547

## Beschreibung

Die vorliegende Erfindung betrifft Polyvinylalkohol Gele, insbesondere Hydrogele, die für den Einsatz im Organismus geeignet sind und am Ort der Anwendung (in situ) aus einer viskosen Flüssigkeit mittels partieller Kristallisation und Netzwerkbildung gebildet werden.

### Stand der Technik

Polyvinylalkohol (PVA), der bsw. durch Hydrolyse von Polyvinylacetat erhalten werden kann, ist biologisch fast vollständig abbaubar und bei erhöhter Temperatur gut wasserlöslich. Hydrogele auf Basis von PVA können mit der Konsistenz von biologischem Gewebe und Knorpel hergestellt werden und weisen im lebenden Organismus eine herausragende Stabilität und Biokompatibilität auf, welche einerseits im hohen Wassergehalt dieser Gele und andererseits im Makromolekül selbst begründet ist, das vom Organismus infolge der zahlreichen Hydroxyl Gruppen ähnlich wie Wasser wahrgenommen wird. Daher sind PVA Gele (PVAG) für Anwendungen im lebenden Organismus geradezu prädestiniert, insbesondere PVAG die ohne chemische Vernetzung, ohne Strahlenvernetzung und ohne Beihilfe von problematischen Chemikalien hergestellt werden.

Bei den bisherigen Verfahren zur Herstellung von solchen PVAG wird in einem ersten Schritt bei erhöhten Temperaturen von bsw. 120°C eine Lösung von PVA hergestellt, die auf Raumtemperatur abgekühlt in eine Form gegossen werden kann. Nachfolgend werden verschiedene Verfahren zur Gelbildung angewandt, wobei die PVA Lösung mindestens einmal eingefroren und dann wieder aufgetaut wird (freeze/thaw). Typischerweise ist das Lösungsmittel Wasser, weisen die PVA Lösungen eine Konzentration Cp von PVA im Bereich von 5 - 15% auf und werden sie mit Abkühlraten von etwa 0.1°C/min auf Temperaturen von rund -15 bis -30°C abgekühlt, etwa 1 - 24h bei dieser Temperatur belassen und dann mit etwa 0.1°Clmin wieder aufgetaut. Nach einem solchen Zyklus sind die PVAG opak und sehr weich. Sie können bereits bei Berührung beschädigt werden, die Festigkeit sm eines PVAG mit Cp = 15% bsw. liegt im Bereich von 0.04MPa und der E-Modul E im Bereich von 0.01 MPa. Durch Wiederholung der freeze/thaw Behandlung werden die mechanischen Eigenschaften kontinuierlich verbessert, nach 10 Zyklen bsw. liegt die Festigkeit sm im Bereich von 1 MPa und der E-Modul im Bereich von 0.1 MPa. Nach weiteren Zyklen werden die mechanischen Eigenschaften nur noch geringfügig verbessert. Solche PVAG sind im Stand der Technik vielfach beschreiben, bsw. von F. Yokoyama et al in Colloid & Polymer Science (1986), 264, Seiten 595 - 601.

Ein modifiziertes Verfahren ist in US 4734097 beschrieben, wobei ausgehend von einer wässrigen PVA-Lösung z.B. in Beispiel 3 mit Cp = 8% nur ein freeze/thaw Zyklus angewandt wird und die PVA-Wasser Mischung im gefrorenen Zustand durch Einsatz eines Vakuums während 10 Stunden auf eine Konzentration Cp von 42% dehydriert wird. Nach dem Auftauen wurde dann ein weissliches opakes Gel mit einer Festigkeit sm von 0.5MPa erhalten, das für den Einsatz als künstliches Gewebe im menschlichen Körper vorgeschlagen wurde. Mit diesem Verfahren hergestellte PVAG wurden für eine Vielzahl von Anwendungen zum Patent angemeldet, bsw. für künstliche Organe und Membranen in EP 0107055 (artificial organs or membrandes for medical use), als dermale Gele in EP 0095892 (wound-covering materials), für den Einsatz als Kühlmedium in EP 0070986 (gel for use as cooling-medium), als Isolations-Gel für tiefe Temperaturen in JP 57190072, als Phantom für die NMR Diagnose in GB 2209401 (phantoms for NMR diagnosis), oder als Golf Ball Füllung in GB 2182571 (golf ball cores).

Ein weiteres modifiziertes Verfahren ist in US 6231605 beschrieben, wobei ausgehend von einer wässrigen PVA-Lösung z.B. in Beispiel 1 mit Cp = 15% zuerst drei freeze/thaw Zyklen bei -20°C angewandt wurden, wonach das erhaltene Gel in Wasser eingelegt und somit gequollen wurde. Das Gel war in diesem Zustand transparent, aber so schwach, dass es seine Form ausserhalb von Wasser nicht aufrechterhalten konnte. Darauf wurde das gequollene Gel noch 2x einer freeze/thaw Behandlung unterzogen und resultierte dann ein opakes elastisches Gel mit einem E-Modul von rund 0.4MPa. Solche Gele wurden ebenfalls als Gewebe Ersatz im menschlichen Körper vorgeschlagen bsw. für Herzklappen, Gefässe, Sehnen, Knorpel, Meniskus Harnröhren.

Bei einem weiteren modifizierten Verfahren in US 4663358 wird die PVA-Lösung mittels Mischungen von Wasser und organischen Lösungsmitteln, insbesondere DMSO, hergestellt und dann bei -20°C gefroren. Anschliessend wird das erhaltene Gel in Wasser gelagert um das DMSO grossteils zu extrahieren, in der Atmosphäre getrocknet und dann unter Vakuum getrocknet, um den Rest an DMSO zu extrahieren. Nach Quellung der Proben in Wasser wurden PVAG erhalten, die eine Transparenz von bis 99% zeigten und Festigkeiten bis 5.6MPa aufwiesen. Solche transparente PVAG wurden für Anwendungen im Bereich Biomedizin und für die Lebensmittelindustrie vorgeschlagen.

Wie erwähnt weisen die genannten Verfahren zur Herstellung von biokompatiblen PVAG die Gemeinsamkeit auf, dass von einer giessfähigen Lösung ausgegangen und mindestens ein freeze/thaw Zyklus angewandt wird. Für eine in situ Gelierung, wo das PVAG innerhalb des Organismus am Ort der Verwendung gebildet wird, sind die bekannten Verfahren offensichtlich nicht geeignet. Für solche Anwendungen ist es notwendig, dass
1. Die PVA-H2O Mischung eine genügend tiefe Viskosität aufweist, die es erlaubt, dass die Mischung z.B. durch eine Kanüle an den Ort der Verwendung im Körper gebracht werden kann und
2. Aus der PVA-H2O Mischung bei Körpertemperatur die Gelbildung abläuft.

Da zunächst keine Verfahren gefunden wurden, die diese Bedingungen erfüllen können, wurden PVAG für biomedizinische Anwendungen ausserhalb des Körpers hergestellt und anschliessend implantiert. Dabei konnten jedoch die Vorteile von PVAG, die in situ gebildet werden, z.B. minimalinvasive Opterationstechniken, nicht genutzt werden. Verfahren zur Herstellung von in situ gelierenden PVAG basieren auf einer chemischen Vernetzung von spritzbaren PVA Lösungen. Solche Verfahren sind in US 2004 166088 A1, US 6602291 und in WO 2004 07069296 A1 beschrieben. Der Nachteil dieser Verfahren besteht darin, dass bei der Vernetzung mittels chemischer Reaktionen Wärme erzeugt wird, welche das umliegende Gewebe schädigen kann und dass verschiedene Nebenprodukte der chemischen Reaktion entstehen, die nicht erwünscht sind, ein schwer kalkulierbares Risiko bedeuten.

Ein weiterer Ansatz zu in situ gelierenden PVA Lösungen ist in US 2004 0171740 A1 und US 2004 0092653 A1 beschrieben. Die Gelbildung erfolgt hierbei auf physikalische Art und Weise durch Netzwerkbildung mittels partieller Kristallisation. Ermöglicht wird dies, indem der Flory Wechselwirkungs Parameter durch Zugabe bsw. von Salzen erhöht wird, wobei allerdings die Salze in hoher Konzentration eingesetzt werden müssen (1.5 bis 6 molar). Somit werden auch bei diesem Verfahren Stoffe in den Organismus eingeführt, die nicht erwünscht sind und zu Komplikationen führen können.

Die vorliegende Erfindung beschreibt eine Lösung zur beschriebenen Problematik, wobei eine spritzbare Polyvinylalkohol und Wasser aufweisende Lösung bei Körpertemperatur durch partielle Kristallisation und Netzwerkbildung zu einem Gel mit erstaunlichen mechanischen Eigenschaften aushärtet. Damit dies geschieht, sind neben PVA und Wasser keine weiteren Stoffe notwendig. Diesbezüglich beschreibt die Erfindung den einfachst möglichen Fall.

### Kurze Beschreibung der Erfindung

Die Erfindung beschreibt eine PVA und Wasser aufweisende Mischung, die im Bereich der Körpertemperatur, insbesondere im lebenden Organismus (in situ) durch partielle Kristallisation ein Netzwerk bildet. Die Mischung ist ausreichend niederviskos, sodass sie minimal invasiv, bsw. durch eine Kanüle an den Ort der Verwendung gebracht werden kann. Die Kinetik der Gelbildung wie auch die mechanischen Eigenschaften des ausgehärteten Gels kann in einem weiten Bereich eingestellt werden, eine merkliche bis deutliche Gelbildung kann nach einigen Minuten erhalten werden. Die Viskosität der Mischung, die Gelierungskinetik und die Endeigenschaften können in weiten Bereichen variiert und an spezifische Erfordernisse angepasst werden. Solche in situ gelierende PVA Mischungen sind als Ersatz von biologischen Geweben und Knorpel geeignet, bsw. können damit defekte Bandscheiben (Hernien) repariert werden.

### Art der eingesetzten PVA

Während bisher für PVAG mit hohen Festigkeiten und E-Moduli vorzugsweise vollhydrolysierte PVA mit höchsten Polymerisationsgraden DP von bis zu etwa 18'000 eingesetzt wurden, wird in dieser Erfindung zumindest teilweise ein gegenteiliger Ansatz angewandt.

Einerseits wird ein erster vorzugsweise vollhydrolysierter PVA1 mit hohem Zahlenmittel des Polymerisationsgrades DPn eingesetzt, andererseits kommt in Kombination mit solchem PVA1 auch ein vorzugsweise vollhydrolysierter PVA2 mit mittlerem und insbesondere niederem DPn im Bereich von etwa 1000 - 40 zum Einsatz. Es ist unmittelbar klar, dass durch die Mischung von PVA1 und PVA2 die Viskosität der Lösung herabgesetzt wird und höhere Konzentrationen Cp mittels Lösungsverfahren verarbeitet werden können. Ein PVA mit DPn = 180 bsw. weist bei Cp = 20% und bei RT eine Viskosität von nur 100 mPas auf und erst bei Cp von etwa 60% wird bei RT die limitierende Viskosität von etwa 30'000mPas erreicht. Bei 100°C kann Cp sogar bis etwa 70% betragen.

Zunächst würde man erwarten, dass durch den Anteil an PVA2 die mechanischen Eigenschaften der resultierenden PVAG reduziert werden, der Vorteil der höheren Lösungskonzentration also dadurch mindestens teilweise wieder aufgehoben wird. Überraschenderweise wurde jedoch ein gegenteiliger Effekt festgestellt. Wird bei gleich bleibendem Cp ein Anteil an PVA1 durch PVA2 ersetzt, resultieren PVAG mit höheren E-Moduli, womit sich der vermeintliche Nachteil als Vorteil herausstellt und zum offensichtlichen Vorteil der höheren Lösungskonzentration Cp hinzukommt. Diese Tendenz bleibt bis zu hohen Substitutionsgraden von PVA1 durch PVA2 gültig. Feste elastische Gele mit hohem Dehnvermögen können bsw. auch noch bei PVA1 : PVA2 = 5 : 95 erhalten werden, während bei Einsatz von PVA2 alleine brüchige Gele mit geringem Dehnvermögen resultieren. Die Wirkung von PVA2 ist damit erklärbar, dass kurzkettige PVA sehr gut, d.h. schnell, vollständig und insbesondere auch bei Temperaturen > 0°C kristallisieren können, wodurch der kristalline Anteil des PVAG erhöht wird. Die bessere Kristallisierbarkeit von PVA2 gegenüber PVA1 ist zumindest teilweise durch die unterschiedliche Kristallisations-Entropie bedingt.

Weiter findet eine Heterokristallisation von PVA1 und PVA2 statt, d.h. die Kristallite, welche die Verknüpfungspunkte des das Gel konstituierenden Netzwerks bilden, weisen sowohl PVA1 als auch PVA2 Makromoleküle auf, wobei die kürzerkettigen PVA2 Makromoleküle die Kristallisation von Segmenten von PVA1 Makromolekülen zu Heterokristalliten induzieren. Es werden somit insgesamt höhere Netzwerkdichten erreicht, d.h. engmaschigere Netzwerke und somit höhere E-Moduli der resultierenden PVAG.

Durch den Einsatz von PVA3, welche Langkettenverzweign mit einem DP dieser Langketten von > 25 ist eine weitere Verbesserung der mechanischen Eigenschaften von PVAG möglich, insbesondere wenn PVA3 in Kombination mit PVA2 und/oder PVA1 eingesetzt wird. Indem die verschiedenen Seitenketten durch Heterokristallisation in verschiedene Kristallite eingebaut werden, entstehen zusätzliche Verknüpfungspunkte des Netzwerks, wobei diese Verknüpfungspunkte dann kovalenter Natur sind.

Während bisher giessbare Konzentrationen Cp im Bereich von 5 bis maximal 30% (abhängig vom DP des PVA) üblich waren, ist nun auch der Bereich von 30 bis etwa 90% zugänglich geworden, wobei auch tiefere Konzentrationen Cp als 30% mit diesem Verfahren möglich sind, was insbesondere bei sehr hohen DPn von PVA1 der Fall ist. Indem ein Anteil an PVA2 eingesetzt wird, der nur einen geringen Beitrag zur Viskosität beisteuert, kann die Viskosität auch bei hohen Konzentrationen Cp tief gehalten werden. Mit der Zunahme von Cp zeigen PVA-Wasser Mischungen bezüglich der Gelbildung im Vergleich zur Gelbildung aus niederkonzentrierten Lösung zunehmend ein ganz anderes Verhalten. Während beim freeze/thaw Verfahren durch den Gefriervorgang dem PVA Wasser entzogen werden muss (Bildung von Phasen von gefrorenem Eis neben Phasen mit sehr hohem Cp bzw. tiefem Wassergehalt) und dadurch die Kristallisation induziert wird, ist bei dem neuen Verfahren Cp von Anfang an hoch und ist die Anwendung von freeze/thaw Zyklen nicht mehr zwingend notwendig. Die Gel- bzw. Netzwerkbildung durch Kristallisation ist auch bei höheren Temperaturen möglich. Bsw. ist bei Cp um 40% die Gelbildung bei Raumtemperatur ebenso effektiv wie bei der Anwendung von freeze/thaw Zyklen. Je höher Cp und der Anteil an PVA2 eingestellt wird, bei umso höherer Temperatur setzt die Gelbildung ein, sodass sogar Temperaturen > RT, insbesondere bei Körpertemperatur zur Anwendung kommen können. Im einfachsten Fall kann eine PVA-Wasser Mischung nach der Formgebung einfach z.B. bei Körpertemperatur gelagert werden, wobei die Gelbildung abläuft. Die Festigkeit der erhaltenen PVAG nimmt mit Cp stetig zu, wobei Werte von bsw. 25MPa und E-Moduli von 30MPa eingestellt werden können und ausserdem vollständige Transparenz erhalten werden kann. Werden solcherart hergestellte PVAG in Wasser eingelegt, findet eine Quellung statt, wodurch Cp und die mechanischen Eigenschaften etwas abnehmen. Der Quellgrad wird dabei wesentlich durch die eingestellte Netzwerkdichte bestimmt und kann durch deren Parameter ebenfalls definiert eingestellt, insbesondere minimiert werden.

### Ausführliche Beschreibung der Erfindung

### PVA1, PVA2, PVA3

Zur Auswahl der geeigneten PVA-Typen werden drei Gruppen von Parametern unterschieden, die Parameter welche die Regularität der PVA betreffen wie Hydrolysegrade H, Gehalt G an 1,2-Glycol, Taktizität, und Anteil an Kurzkettenverzweigungen, die Parameter der Molekulargewichtsverteilung wie DPn (Zahlenmittel), DPw (Gewichtsmittel), sowie die Parameter zur Topologie der Makromoleküle wie Verzweigungsgrade von Langketten und Länge dieser Lang- bzw. Seitenketten.

Bezüglich der ersten Gruppe von Parametern, werden an PVA11, PVA2 und PVA3 dieselben Anforderungen gestellt, welche eine möglichst gute Kristallisierbarkeit der PVA und eine hohe Stabilität der Kristallite ermöglichen. Somit sind Abweichungen von der idealen Struktur [-CH₂-CHOH-]ₙ anteilmässig möglichst gering zu halten.

Der Hydrolysegrad H in mol% von PVA1, PVA2 und PVA3 ist > 98, vorzugsweise > 99, noch bevorzugter > 99.2 noch bevorzugter > 99.4. In einer besonders vorteilhaften Ausführung und insbesondere bei PVA2 ist H > 99.85, vorzugsweise > 99.9, noch bevorzugter > 99.95, am bevorzugtesten > 99.98. Im Bereich dieser hohen Hydrolysegrade hat eine nur geringe Steigerung einen erstaunlich deutlichen Effekt auf die Endeigenschaften des Gels.

Vorteilhaft ist ein Gehalt G an 1,2-Glycol in mol% von < 3, vorzugsweise < 1, noch bevorzugter < 0.5, am bevorzugtesten < 0.2.

Vorteilhaft ist eine Anzahl an Kurzkettenverzweigungen pro Monomereinheit von < 10⁻², vorzugsweise < 10⁻³, noch bevorzugter < 10⁻⁴, am bevorzugtesten < 10⁻⁶.

Weitere Störungen der Regularität wie Carbonylgruppen in der Kette sind ebenfalls unerwünscht, bei üblichen PVA ist ihr Anteil mit typischerweise < 0.02mol% jedoch vernachlässigbar.

Bezüglich der Taktizität wird eine ataktische Konformation gegenüber einer isotaktischen bevorzugt, am bevorzugtesten ist eine syndiotaktische Konformation, bez. ein möglichst hoher Anteil an syndiotaktischen Diaden. Die Taktizität von PVA wird durch die Art der Monomere festgelegt, womit das Vorläufer Polymer, woraus dann das PVA erhalten wird, polymerisiert wird, sowie durch die Reaktionsbedingungen bei dieser Polymerisation, wobei mit abnehmender Temperatur während der Polymerisation der syndiotaktische Anteil zunimmt.

Wird das Vorläufer Polymer aus Vinylacetatderivaten der Art CH₂=CHOCOR polymerisiert, wobei R bsw. H, CH₃, C₃H₇, C₄H₉, CCIH₂, CCl₃, CF₃ C₄HsF₄, C₆H₇F_{6,} oder C₆H₅ sein kann, so nimmt der Anteil an syndiotaktischen Diaden mit dem Volumen der Gruppe R zu (während der 1,2-Glycolgehalt vorteilhaft abnimmt) und die im Vorläufer Polymer erhaltene Taktizität bleibt bei der nachfolgenden Hydrolyse zum PVA erhalten. Bevorzugt sind daher Monomere zur Polymerisation des Vorläufer Polymers wie Vinylacetat, Vinylchloroacetat, Vinyldichloroacetat, Vinylbromoacetat, insbesondere Vinyltrifluoroacetat.

Wird das Vorläufer Polymer aus aliphatischen Vinylsäureestem hergestellt, werden ebenfalls hohe Anteile an syndiotatkischen Diaden erhalten, während die resultierenden PVA ausserdem sehr niedrige 1,2-Glycol Gehalte aufweisen. Beispiele sind Vinylformat, Vinylpropionat, Vinylbutyrat, Vinylpivalat. Mittels Vinylpivalat können auch sehr hohe Molekulargewichte erreicht werden.

Vollhydrolysierte PVA erhalten aus Polyvinylacetat sind auch bei hohen Kristallisationsgraden bei 100°C in Wasser löslich, während vollhydrolysierte PVA deren Vorläufer Polymer aus Vinylacetatderivaten mit voluminöser Gruppe R (z.B. Vinyltrifluoroacetat) oder aus aliphatischen Vinylsäureestern (z.B. Vinylfromat, Vinylpivalat) hergestellt wurden infolge des geringen 1,2-Glycolgehalt und des hohen Anteils an syndiotaktischen Diaden selbst bei 100°C in unlöslicher Form erhalten werden können. Daraus wird die Bedeutung dieser Parameter für die Kristallisierbarkeit und die Stabilität der Kristallite deutlich. Solche PVA sind darum für die vorliegende Erfindung ganz besonders geeignet.

Bezüglich der Molekulargewichtsverteilung werden an PVA1, PVA2 und PVA3 unterschiedliche Anforderungen gestellt. Für bisherige, auf nur einem PVA basierende PVAG wird in der Literatur als eine untere Grenze für den mittleren Polymerisationsgrad DP typischerweise ein Wert von 1000 bis 1500 angegeben, wobei die Art des Mittelwertes (DPn, DPw, DPv) meist nicht spezifiziert wird. Die Polydispersität P = DPw/DPn von aus Polyvinylacetat durch Verseifung erhaltenem PVA liegt im Bereich von etwa 2 - 2.5, womit eine Umrechung dieser Mittelwerte und eine Interpretation möglich sind. Bei DPn um 500 werden nach bisherigen Methoden bröckelige und brüchige PVAG erhalten. Dies ist dadurch verständlich, dass die Makromoleküle nicht lang genug sind, um effektive Verbindungen zwischen Kristalliten zu bilden. Somit können die Kristallite leicht aneinander abgleiten und es wird kaum Festigkeit erhalten. Mit abnehmendem DPn nimmt diese bröckelige Konsistenz weiter zu. Eine Voraussetzung für effektive Verbindungen ist, dass mindestens zwei Segmente eines Makromoleküls in mindestens zwei Kristallite eingebaut werden. Mit zunehmendem DPn nimmt die Zahl der Kristallite, woran verschiedene Segmente eines PVA Makromoleküls beteiligt sind zu und werden mechanisch stabilere und zunehmend elastische Netzwerke erhalten, weshalb bei bisherigen PVAG möglichst hohe DP vorteilhaft sind. Die Tatsache, dass bei tiefen DP nur bröckelige und brüchige PVAG erhalten werden, mag ein Grund gewesen sein, warum bisher tiefe DP für PVAG nicht in Betracht gezogen wurden.

Für PVA1 und PVA3 werden für die vorliegende Erfindung PVA mit DPn > 1000, vorzugsweise > 1500, noch bevorzugter > 2000, am bevorzugtesten > 3000 eingesetzt. Die obere Grenze ist durch den Stand der Technik zur Herstellung von höchstmolekularen PVA gegeben, wobei gegenwärtig PVA mit DP bis etwa 18'000 herstellbar sind.

Für PVA2 werden PVA mit vergleichsweise tiefen DP eingesetzt, einerseits um die Viskosität der Lösungen und Schmelzen zu reduzieren, andererseits um Kristallisation auch bei Temperaturen > 0°C zu ermöglichen sowie hohe Kristallisationsgeschwindigkeiten, Kristallinitätsgrade und hohe Netzwerkdichten zu erhalten. PVA2 wird für die vorliegende Erfindung mit DPn im Bereich von etwa 40 - 1000, vorzugsweise 50 - 900, noch bevorzugter 70 - 800, am bevorzugtesten 75 - 700 eingesetzt. Die untere Grenze ist durch die Stabilität der durch PVA2 gebildeten Kristallite gegeben (wobei durch Heterokristallisation Segmente von PVA1 und PVA3 eingebaut sind). Die Lamellendicke dieser Kristallite ist bei tiefen DP direkt proportional zum DP und die Stabilität gegenüber Temperatur und Lösungsmittel (Wasser) nimmt mit der Lamellendicke zu. Bei hohen DP nimmt die Lamellendicke meist wieder ab, da dann die Makromoleküle nicht mehr in der voll gestreckten Konformation kristallisieren, sondern vorzugsweise eine Rückfaltung stattfindet und tiefere Lamellendicken resultieren. Somit kann durch die Wahl geeigneter DP von PVA2 bei hohen Hydrolysegraden auch ein positiver Effekt bezüglich der Stabilität der Kristallite erhalten werden, was insbesondere für in vivo Anwendungen von PVAG bedeutsam ist. Der diesbezüglich optimale DP Bereich liegt bei etwa 75 - 700.
Betreffend der Polydispersität P = DPw/DPn der drei PVA Typen, insbesondere bei PVA2, ist P < 5 bevorzugt, noch bevorzugter < 3, am bevorzugtesten < 2.

Bezüglich der Topologie sind übliche PVA vorwiegend linear, Langkettenverzweigungen treten bei üblichen PVA wenn überhaupt, dann selten auf. Nahezu vollständige oder vollständige Linearität wird bei kurzkettigen PVA2 bevorzugt, wobei diese Bedingung praktisch immer erfüllt ist, während langkettige PVA1 nicht notwendigerweise möglichst linear sein müssen, ein Anteil an Langkettenverzweigungen kann bei PVA1 sogar vorteilhaft sein, wenn die Länge dieser Seitenketten einen DP > 40 aufweist. Bei PVA3 hingegen ist ein nennenswerter Anteil an Langkettenverzweigungen für dessen Funktionalität das entscheidende Eigenschaftsmerkmal, wobei für die Polymerisationsgrade DP dieser Lang- bzw. Seitenketten dieselben Anforderungen gelten wie für die DP von PVA2. Somit ist es möglich, dass die verschiedenen Seitenketten eines Makromoleküls PVA3 in verschiedene Kristallite eingebaut werden können (zusammen mit PVA1 und/oder PVA2), wodurch eine zusätzliche Vernetzung dieser Kristallite resultiert, also höhere Netzwerkdichten erhalten werden. PVA vom Typ PVA3 sind gegenwärtig kommerziell nicht erhältlich. Von PVA erhalten aus Polyvinylbenzoat ist jedoch bekannt, dass bei geeigneten Reaktionsbedingungen bei der Polymerisation von Vinylbenzoat Langkettenverzweigungen erhalten werden können. Eine weitere Möglichkeit zur Herstellung von PVA3 kann durch Aufpfropfen von PVA vom Typ PVA2 auf PVA vom Typ PVA1 erfolgen, wobei die Anzahl und Länge der Seitenketten eingestellt werden kann.

### Mischungen

In einer vorteilhaften Ausführung liegt der Anteil an PVA1 bezogen auf PVA1 + PVA2 in Gew.% im Bereich 3 - 60 , vorzugsweise 5 - 50, noch bevorzugter 7 - 40, am bevorzugtesten 8 - 35. Durch die Wahl des Anteils an PVA1 wird einerseits die Viskosität der Lösung eingestellt, wobei die Viskosität mit dem Anteil an PVA1 zunimmt. Andererseits wird mit zunehmendem Anteil an PVA2 die Viskosität gesenkt und die Kristallisation gefördert, d.h. sie kann bei zunehmend höheren Temperaturen stattfinden und schneller. Der Anteil an PVA1 hängt auch davon ab, welcher Typ von PVA1 eingesetzt wird. Z.B. bei hohen Polymerisationsgraden von PVA1 wird der Anteil an PVA1 mit bsw. 8% tief angesetzt.

Ein hoher Anteil an PVA2 bezogen auf PVA1 + PVA2 ist zur Einstellung von hohen E-Moduli vorteilhaft, wobei erstaunlicherweise immer noch sehr hohe Dehnungen erhalten werden.

Der Anteil an PAV3 bezogen auf PVA1 + PVA2 + PVA3 in Gew.% liegt im Bereich 1 - 80, vorzugsweise 2 - 60, am bevorzugtesten 3 - 50.

Der Anteil an PVA bezogen auf PVA und Quellungsmittel in Gew.% liegt im Bereich 5 - 80, vorzugsweise 10 - 70, noch bevorzugter von 15 - 60. am bevorzugtesten von 20 - 50.

In einer vorteilhaften Ausführung liegt die dynamische Viskosität bei 70°C der Mischung in MPas bei < 30'000, vorzugsweise < 20'000, noch bevorzugter < 15'000, am bevorzugtesten < 10'000.

### Aufbereitung und Gelbildung

Zur Herstellung erfindungsgemässer PVAG werden die Komponenten PVA1, PVA2 und gegebenenfalls PVA3 in eine wässrige Lösung überführt. Vorzugsweise wird für den Lösevorgang eine Ringer Lösung eingesetzt. Dabei können die Komponenten separat oder gemeinsam gelöst werden und für die Herstellung der Lösung können die bekannten Standardverfahren eingesetzt werden. Wichtig ist eine gute Durchmischung der Komponenten, wozu verschiedene bekannte Mischverfahren für viskose Lösungen eingesetzt werden können. Die Eigenschaft der Gelierbarkeit unter in situ Bedingungen (um 37°C, im Körper), die Kinetik der Gelierung und die Endeigenschaften des Gels werden durch die Art der eingesetzten Komponenten, ihr Verhältnis und durch den Wassergehalt eingestellt.

### Eigenschaften

Die erfindungsgemässen Gele zeichnen sich durch die einfache Verarbeitbarkeit, die schnelle Gelbildung, die guten mechanischen Eigenschaften und durch eine hohe Stabilität aus. Die Möglichkeit auf die verschiedenen Eigenschaften gezielt Einfluss zu nehmen und spezifische Eigenschaftskombinationen zu erhalten liegt darin begründet; dass gegenüber anderen Lösungen ein wesentlicher neuer Freiheitsgrad durch das kurzkettige PVA2 und dessen Parameter (Hydrolysegrad, DPn, minimaler DP, Aufreinigung) zur Verfügung steht.

In einer vorteilhaften Ausführung wird nach einer Stunde mindestens 10% des E-Moduls und/oder der Festigkeit des ausgehärteten Zustandes errecht, vorzugsweise mindestens 25%, noch bevorzugter mindestens 40%, am bevorzugtesten mindestens 50%. Mit zunehmendem Anteil an PVAG2 steigt die Gelbildungsgeschwindigkeit, sowie mit dem Hydrolysegrad von PVA1 und insbesondere von PVA2.

In einer vorteilhaften Ausführung weisen die Gele einen E-Modul und/oder eine Festigkeit in MPa im Zugmodus und/oder im Kompressionsmodus von 0.1 - 25 auf, vorzugsweise von 0.25 - 25, noch bevorzugter von 0.5 - 25, am bevorzugtesten von 0.75 - 25. Die Wirkung der verschiedenen Parameter auf die mechanischen Eigenschaften ist aus Tabelle 1 ersichtlich.

Von grosser Bedeutung ist weiter die Stabilität der Gele in der Anwendung. Klassische freeze/thaw PVA Gele weisen eine Schwäche betreffend der Stabilität auf. Da die Gelbildung bei sehr viel tieferen Temperaturen (<< 0°C) abläuft als bei der Einsatztemperatur (37°C), ist ein Anteil der Kristallite bei der Einsatztemperatur nicht stabil. Sie lösen sich wieder auf und Makromoleküle gehen in Lösung. Da bei den erfindungsgemässen Gelen die Einsatztemperatur mit der Gelbildungstemperatur identisch ist, findet dieser Verlust an Polymer, wodurch das Gel geschwächt wird, nicht statt. Ausserdem ist ein möglichst geringer Verlust an Polymer für in vivo Anwendungen vorteilhaft. Andererseits können Polymere trotzdem in Lösung gehen, wenn sie zu kurz sind um stabile Kristallite zu bilden oder wenn der Hydrolysegrad zu tief ist oder wenn andere Abweichungen von der regulären Struktur vorliegen. Solche ungeeignete Makromoleküle können eliminiert werden, wenn die Polymere zuvor gelöst und unter geeigneten Bedingungen ausgefällt werden, wobei die ungeeigneten Makromoleküle in der Lösung verbleiben. Eine andere Möglichkeit besteht darin, dass die Polymere möglichst vollständig nachhydrolysiert werden und zu kurze Makromoleküle bsw. durch Dialyse herausgefiltert werden. Die hierzu angewandten Verfahren sind weiter hinten beschrieben (Beispiele 2 - 12, Analysen) und die Auswirkung auf den Verlust an Polymer nach 14 Tagen in destilliertem Wasser, V14 ist aus Tabelle 1 ersichtlich. In einer bevorzugten Ausführung liegt der Polymerisationsgrad der kleinsten Makromoleküle der Molekulargewichtsverteilung von PVA1 und PVA2 bei > 40, vorzugsweise > 50, noch bevorzugter > 60, am bevorzugtesten > 70.

In einer vorteilhaften Ausführung ist V14 in % < 7, vorzugsweise < 4, noch bevorzugter < 3, am bevorzugtesten < 2.

Im Allgemeinen wird der Wassergehalt der gebildeten Gele erhöht, wenn sie in Wasser eingelegt werden (Quellung). Dieselben Massnahmen jedoch, die zu einer Reduktion von V14 führen reduzieren auch den Quellgrad deutlich, sodass der Wassergehalt nach Quellung nur noch geringfügig grösser ist als zuvor. Somit können auch Gele mit herausragenden mechanischen Eigenschaften erhalten werden. Der Quellgrad kann weiter minimiert werden, wenn die Gelbildung in einer Ringer Lösung stattfindet und die Quellung dann in einer Ringer Lösung durchgeführt wird. Somit sind die Bedingungen auch sehr nahe bei den Bedingungen im Organismus, wo das Gel zum Einsatz kommt.

### Quellungsmittel

Als Quellungsmittel werden hier Quellungsmittel im engeren Sinne, als auch Lösungsmittel bezeichnet. Das wichtigste Quellungsmittel ist Wasser und Wasser wird in den meisten Fällen als alleiniges Quellungsmittel eingesetzt, oder in Mischungen mit anderen Quellungsmitteln, daneben kommen jedoch die im Stand der Technik beschriebenen Lösungs- und Quellungsmittel und Mischungen davon in Frage wie bsw. DMSO, Dimethylformamid, Acetamid oder Polyole wie bsw. Glycerin, Erythritol, Xylitol, Sorbitol, Mannitol, Galactitol, Tagatose, Lactitol, Maltitol, Maltulose, Isomalt, Methylenglycol, Ethylenglycol, Diethylenglycol, Triethylenglycol, Propylenglycol, Butandiol, Pentandiol, Hexantriol. Das Quellungsmittel kann auch einen Anteil an Salz aufweisen (physiologische Salzlösung, Ringer Lösung).

### Weitere Polymere

Neben PVA kann das PVAG zur Modifikation der Eigenschaften und für spezifische Anwendungen weitere Polymere enthalten, bsw. synthetische Polymere wie bsw. Polycarbonate, Polyacrylate und Polymethacrylate, Polyethylenglycole, Polythylenoxide, Polyvinylpyrrolidone, Polycaprolactone oder Polymere natürlichen Ursprungs wie bsw. Hydrokolloide und Polysaccharide, insbesondere Stärke und Stärke Derivate.

### Zusatzstoffe

Als Zusatzstoffe werden einfache Füllstoffe und funktionale Füllstoffe bzw. Wirkstoffe bezeichnet.

### Anwendungen

Da die erfindungsgemässen PVAG mit einem weiten Range von mechanischen Eigenschaften erhalten werden können, sind eine ganze Reihe von Anwendungen möglich. Einerseits können bisherige PVA Gele in allen Anwendungen vorteilhaft ersetzt werden, da die neuen Gele sehr viel einfacher herzustellen sind (Giessbarkeit, Gelbildungstemperatur > 0°C, Stabilität) und mindestens gleich gute mechanische Eigenschaften aufweisen. Andererseits eröffnen die neuen Gele, insbesondere als in situ Gele ausserordentlich interessante Anwendungen im Bereich der Biomedizin (z.B. Tissue und Scaffold Engineering) und insbesondere in der Orthopädie. Gewebe, Gele, Knorpel, insbesondere Sehnen, Bänder Gelenk Oberflächen, Menisci, Mervenummantelungen, Harnröhren, Herzklappen, Gelkörper der Bandscheiben (Nucleus) können ersetzt oder repariert werden. Im Bereich der Bandscheiben kommen insbesondere folgende spezifische Anwendungen in Frage: Total Disc Replacement, Nulceoplasty, Facet Replacement, Segment Replacement, Vertebroplasty, Kyphoplasty, Lordoplasty. Weitere Anwendungen liegen im Bereich der kosmetischen bzw. plastischen Chirurgie, wo die Gele eine Alternative zu Silikon Implantaten darstellen.

### Beispiele

### Beispiel 1

PVA1: 99.4% hydrolysiert, DPn = 2200, PVA2: 98.5% hydrolysiert, DPn = 180. 10g PVA1 wurde mit 100ml H2O im Kolben unter Rühren mit einem Magnetrührer bei 100°C während 30min gelöst. Von dieser homogenen Lösung wurde 35g in einen Brabender Kneter transferiert. Die Temperatur des Kneter lag bei 90°C und die Drehzahl wurde auf 180upm eingestellt. Zu der Lösung in der Knetkammer wurde 30g PVA2 in der Form von Pulver zudosiert. Nach 4min Mischzeit wurde eine homogene Mischung erhalten. Der Wassergehalt dieser Mischung lag bei 49% (Wasser bezogen auf PVA und Wasser) und der Anteil an PVA1 bezogen auf PVA1 und PVA2 lag bei 9.6%. Von der Mischung wurde 5ml in eine Spritze eingezogen, die vorgängig bei 40°C temperiert worden war. Nach 3min wurde die PVA Lösung durch eine Injektionsnadel IN1 von 5cm Länge und 1 mm Innendurchmesser in eine auf 40°C temperierte Petrischale ausgetragen. Die Viskosität war tief genug, dass die Lösung von Hand mit geringem Druck durch die Injektionsnadel ausgetragen werden konnte. Nach etwa 3min konnte eine deutliche Gelbildung festgestellt werden. Der Rest der Mischung wurde zur Herstellung eines Films von 0.5mm Dicke verwendet, womit Zugversuche und Stabilitätsuntersuchungen durchgeführt wurden.

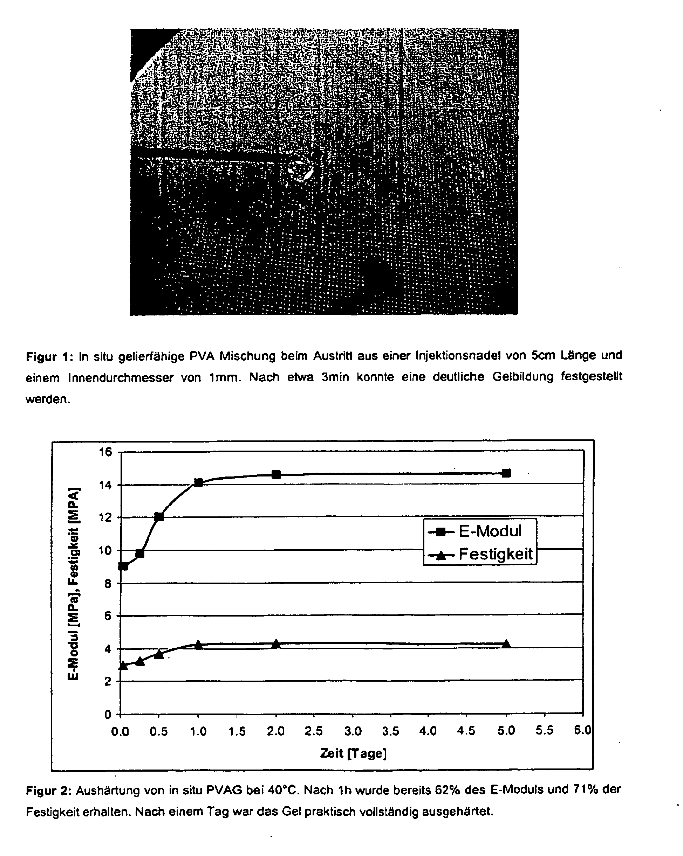

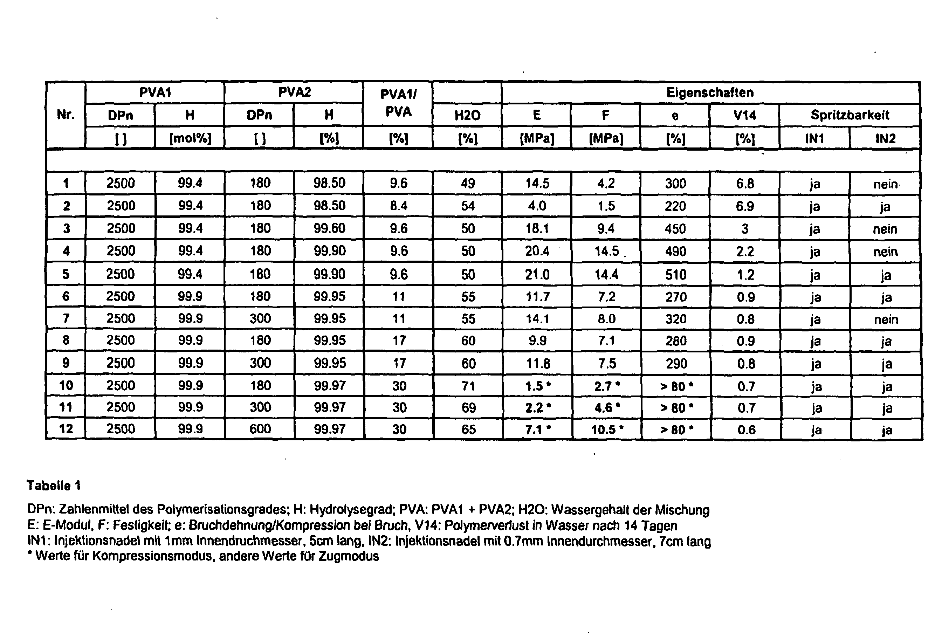

Der Austrag der PVA Lösung durch die Injektionsnadel ist in Bild 1 dargestellt. In Bild 2 ist der Aushärtungsvorgang in Funktion der Zeit anhand des E-Moduls und der Festigkeit wiedergegeben. Die Bruchdehnung variierte nur gering mit der Aushärtungszeit und lag bei rund 300%. Der E-Modul im Zugversuch lag nach Aushärtung bei 14.5MPa und die Festigkeit bei 4.2MPa. Die Stabilitätsuntersuchung ergab einen Verlust an Polymer V14 von 6.8%.

### Beispiele 2 - 12

Bei den Beispielen 2 - 12 wurde ein vergleichbares Vorgehen wie in Beispiel 1 angewandt. Variiert wurde der Anteil an PVA1, der Wassergehalt der Mischung und die eingesetzten PVA Typen. Die entsprechenden Daten und die Eigenschaften der erhaltenen Gele sind in der Tabelle 1 aufgeführt. Bei den Beispielen 4 - 12 wurde ausserdem die Lösung von PVA mit einer Ringer Lösung anstelle von reinem Wasser hergestellt. Die Hydrolysegrade H > 99.4mol% von PVA1 und die Hydrolysegrade H > 98.5mol% von PVA2 wurden durch Nachhydrolysierung erhalten. Bei Beispiel 5 wurde das PVA2 durch Fällung einer 20%igen Lösung bei 5°C aufgereinigt, wobei selektiv die besser kristallisierenden Makromoleküle ausgefällt wurden. Die Stabilitätsuntersuchung zeigte folglich einen deutlich verbesserten Verlust an Polymer V14 von nur noch 1.2%. Die Resultate in Tabelle 1 zeigen, dass durch eine Erhöhung des Hydrolysegrades von PVA1 und PVA2 ebenfalls deutlich verbesserte Stabilitätswerte V14 erhalten werden konnten. Ausserdem konnten auch bei den höchsten Wassergehalten noch sehr gute mechanische Eigenschaften erhalten werden.

Die Beispiele 10 und 11 eignen sich gut für die Wiederherstellung von defekten Bandscheiben. Dabei wird bei noch intaktem Annulus eine Gelfestigkeit in Kompression von 1-2MPa und ein E-Modul in Kompression von etwa 0.5 - 2MPa benötigt, das Gelvolumen liegt bei rund 5ml und die Temperatur der injizierten PVA Lösung sollte unterhalb von etwa 55°C liegen, die Gelierung bei 37°C ablaufen und die Viskosität der PVA Lösung sollte tief genug sein, dass sie durch eine Kanüle von etwa 2mm Innendurchmesser injiziert werden kann. Bei nicht mehr intaktem Annulus (z.B. beim Vorliegen einer Hernie) liegt die geforderte Gelfestigkeit bei etwa 2 - 5MPa, während die weiteren Bedingungen gleich bleiben mit Ausnahme des Durchmessers der Kanüle, der bis 6mm gross sein kann. Das Beispiel 12 zeigt Eigenschaften, wie sie für Knorpel benötigt werden. Die weiteren Beispiele zeigen, dass auch höhere Werte als gefordert möglich sind, also ein weiter Bereich von mechanischen Eigenschaften zugänglich ist. Die aufgeführten Beispiele sind optimierte Gele, selbstverständlich ist es möglich auch Gele mit tieferen E-Moduli und Festigkeiten zu erhalten.

### Analysen

Hydrolysegrad H: Durch Bestimmung der Esterzahl in Anlehnung an DIN 53401.

Polymerisationsgrad: Bestimmung der Grenzviskosität nach C.A. Finch (Polyvinyl Alcohol, Properties and Applications, 1973) und Korrelation der erhaltenen Grenzviskositäten mit den Grenzviskositäten von Polyvinylstandards mit bekanntem DPn.

Hydrolysierung: Verseifung von Polyvinylalkohol Typen Mowiol 3-98, 4-98, 6-98, 56-98 und 66-100 mit NaOH bei 25°C bei anfänglichem pH von 12 nach C.A. Finch (Polyvinyl Alcohol, Properties and Applications, 1973). Zunehmende Hydrolysegrade wurden durch zunehmende Reaktionszeiten erhalten, wobei die Reaktion durch Senkung des pH auf 7 gestoppt wurde. Die Reaktionsprodukte wurden dann mit Aceton ausgefällt (Aceton : Wasser = 1 : 1) in Wasser: Aceton = 2 : 1 in einer Nutsche gewaschen, getrocknet und pulverisiert (Schlagmühle), oder (Versuche 10 - 12) im Anschluss an die Reaktion zuerst während 48h dialysiert (Molecular Weight Cut Off = 3500, Spectra/Por, Spectrum), dann mit Aceton ausgefällt, getrocknet und pulverisiert. Durch die Dialyse konnten kürzere Polymerketten, die keine stabilen Kristallite zu bilden vermögen abgetrennt werden, wodurch die Stabilität wie auch die mechanischen Eigenschaften verbessert werden konnten.

Mechanische Eigenschaften: Die mechanischen Eigenschaften im Zugmodus und im Kompressionsmodus wurden mit einem Instron Tensile Tester 5542 durchgeführt. Die Proben für die Zugversuche wurden aus gepressten Filmen von 0.5mm Dicke herausgestanzt und wiesen eine Probenlänge von 13mm und eine Breite von 2mm auf. Die Dehngeschwindigkeit betrug 10cm/min. Die Proben für die Kompressionsversuche wurden aus gegossenen Platten der Dicke 0.5cm als Rundkörper mit 0.5cm Durchmesser herausgestanzt. Die Kompressionsgeschwindigkeit lag bei 0.5cm/min. Die erhaltenen Messwerte sind jeweils Mittelwerte von 5 einzelnen Messungen.

Stabilitätsuntersuchung: Filme der Dicke 0.5mm und der Fläche von 1cm2 mit einem Wassergehalt W0 und einem Gewicht M0 wurden während 14 Tagen im Überschuss Wasser bei Raumtemperatur gelagert. Danach wurden die Filme durch Abwischen mit saugfähigem Papier vom Oberflächenwasser befreit, gewogen (Gewicht M1) und ihr Wassergehalt (W1) bestimmt. Der Massenverlust in % nach 14 Tagen, V14 wurde sodann erhalten als 100(1 - M1(1-W1)/(M0(1-W0))).

Spritzbarkeit: Es wurden zwei Arten von Injektionsnadeln verwendet: IN1 hatte einen Innendurchmesser von 1 mm und eine Länge von 5cm, IN2 hatte einen Innendurchmesser von 0.7mm und eine Länge von 7cm. Die Spritzbarkeit wurde als positiv beurteilt, wenn bei 40°C während 1 min bei manuellem Kraftaufwand mindestens 2g PVA Lösung ausgetragen werden konnten.

Viskosität: Dynamische Viskositäten wurden mit einem Kegel-Platte Viskosimeter US 200 von Paar Physica bei 70°C gemessen.

## Patentansprüche

1. Spritzbares Polyvinylalkohol Gel, **dadurch gekennzeichnet, dass**
a) das Gel aus einer Lösung bei 0 - 100°C gebildet wird, die notwendigerweise nur Polyvinylalkohol und Wasser aufweist; und
b) das Gel mindestens einen Polyvinylalkohol des Typs PVA1 und mindestens einen Polyvinylalkohol des Typs PVA2 aufweist; und
c) der Polymerisationsgrad DPn von PVA1 > 1000 ist; und
d) der Polymerisationsgrad DPn von PVA2 im Bereich von 50 - 1000 liegt; und
e) der Hydrolysegrad von PVA1 und PVA2 in mol% > 98 ist.

2. Spritzbares Polyvinylalkohol Gel, **dadurch gekennzeichnet, dass**
a) das Gel aus einer Lösung bei 0 - 100°C gebildet wird, die notwendigerweise nur Polyvinylalkohol und Wasser und mindestens einen Zusatzstoff in Form eines Füllstoffs oder Wirkstoffs und/oder mindestens ein Quellungsmittel und/oder mindestens ein weiteres Polymer aufweist; und
b) das Gel mindestens einen Polyvinylalkohol des Typs PVA1 und mindestens einen Polyvinylalkohol des Typs PVA2 aufweist; und
c) der Polymerisationsgrad DPn von PVA1 > 1000 ist; und
d) der Polymerisationsgrad DPn von PVA2 im Bereich von 50 - 1000 liegt; und
e) der Hydrolysegrad von PVA1 und PVA2 in mol% > 98 ist.

3. Spritzbares Polyvinylalkohol Gel nach Anspruch 2, **dadurch gekennzeichnet, dass** das weitere Polymer ausgewählt ist aus der Gruppe, bestehend aus Polycarbonaten, Polyacrylaten und Polymethacrylaten, Polyethylenglycolen, Polythylenoxiden, Polyvinylpyrrolidonen, Polycaprolactonen, Hydrokolloiden und Polysacchariden.

4. Spritzbares Polyvinylalkohol Gel nach einem der vorangehenden Ansprüche, **dadurch gekennzeichnet, dass** der Hydrolysegrad von PVA2, gegebenenfalls auch der Hydrolysegrad von PVA1 in mol% > 99,85, vorzugsweise > 99,9, noch bevorzugter > 99,95, am bevorzugtesten > 99,98 ist.

5. Spritzbares Polyvinylalkohol Gel, nach einem der vorangehenden Ansprüche, **dadurch gekennzeichnet, dass** die kleinsten Makromoleküle der Molekulargewichtsverteilungen von PVA1 und PVA2 einen Polymerisationsgrad von > 40 aufweisen.

6. Spritzbares Polyvinylalkohol Gel nach einem der vorangehenden Ansprüche, **dadurch gekennzeichnet, dass** die Gelbildung im Bereich der Körpertemperatur unter in situ Bedingungen erfolgt.

7. Spritzbares Polyvinylalkohol Gel nach einem der vorangehenden Ansprüche, **dadurch gekennzeichnet, dass** das Gel bei Raumtemperatur in Kompression und/oder im Zugversuch einen E-Modul im Bereich von 0,1 - 25MPa aufweist.

8. Spritzbares Polyvinylalkohol Gel nach einem der vorangehenden Ansprüche, **dadurch gekennzeichnet, dass** das Gel nach 1h mindestens 10% des E-Moduls und/oder der Festigkeit des ausgehärteten Zustandes aufweist.

9. Spritzbares Polyvinylalkohol Gel, nach einem der vorangehenden Ansprüche, **dadurch gekennzeichnet, dass** ein Film von etwa 0,5mm Dicke in destilliertem Wasser gequollen bei Raumtemperatur nach 14 Tagen einen Verlust an Polymer in % von < 7, vorzugsweise < 3 aufweist.

10. Verwendung eines spritzbaren Polyvinylalkohol Gels nach einem der vorangehenden Ansprüche als Ersatz in Anwendungen bisheriger Gele und Polyvinalalkohol Gele, insbesondere im Bereich der Biomedizin, im Bereich des Tissueund Scaffold-Engineering, in der Orthopädie und im Bereich der plastischen Chirurgie.

11. Verwendung eines spritzbaren Polyvinylalkohol Gels nach einem der vorangehenden Ansprüche im Bereich des Ersatz und der Reparatur von Bandscheiben, insbesondere des Nucleus.

## Claims

1. An injectable polyvinyl alcohol gel **characterized in that**
a) the gel is formed from a solution at 0 - 100 °C, which necessarily comprises only polyvinyl alcohol and water; and
b) the gel comprises at least one polyvinyl alcohol of type PVA1 and at least one polyvinyl alcohol of type PVA2; and
c) the degree of polymerization DPn of PVA1 is > 1000; and
d) the degree of polymerization DPn of PVA2 is in the range of 50 -1000; and
e) the degree of hydrolysis of PVA1 and PVA2 in mol% is > 98.

2. An injectable polyvinyl alcohol gel **characterized in that**
a) the gel is formed from a solution at 0 - 100 °C, which necessarily comprises only polyvinyl alcohol and water and at least one additive in the form of a filler or active ingredient and/or at least one swelling agent and/or at least one additional polymer; and
b) the gel comprises at least one polyvinyl alcohol of type PVA1 and at least one polyvinyl alcohol of type PVA2; and
c) the degree of polymerization DPn of PVA1 is > 1000; and
d) the degree of polymerization DPn of PVA2 is in the range of 50 - 1000; and
e) the degree of hydrolysis of PVA1 and PVA2 in mol% is > 98.

3. An injectable polyvinyl alcohol gel according to claim 2, **characterized in that** the additional polymer is selected from the group consisting of polycarbonates, polyacrylates and polymethacrylates, polyethylene glycols, polyethylene oxides, polyvinylpyrrolidones, polycaprolactones, hydrocolloids and polysaccharides.

4. An injectable polyvinyl alcohol gel according to any of the previous claims, **characterized in that** the degree of hydrolysis of PVA2, optionally also the degree of hydrolysis of PVA1, in mol% is > 99.85, preferably > 99.9, more preferably > 99.95, most preferably > 99.98.

5. An injectable polyvinyl alcohol gel according to any of the previous claims, **characterized in that** the smallest macromolecules of the molecular weight distributions of PVA1 and PVA2 have a degree of polymerization of > 40.

6. An injectable polyvinyl alcohol gel according to any of the previous claims, **characterized in that** gel formation occurs in the range of body temperature under in situ conditions.

7. An injectable polyvinyl alcohol gel according to any of the previous claims, **characterized in that** the gel has at room temperature in compression and/or in the tensile test an E modulus in the range of 0.1 - 25 MPa.

8. An injectable polyvinyl alcohol gel according to any of the previous claims, **characterized in that** the gel has after 1 h 10 % of the E modulus and/or the strength of the cured state.

9. An injectable polyvinyl alcohol gel according to any of the previous claims, **characterized in that** a film of a thickness of approximately 0.5 mm swelled in distilled water at room temperature after 14 days has a loss of polymer in % of < 7, preferably < 3.

10. The use of an injectable polyvinyl alcohol gel according to any of the previous claims as replacement in applications of present gels and polyvinyl alcohol gels, in particular in the area of biomedicine, in the area of tissue and scaffold engineering, in orthopedics, and in the area of plastic surgery.

11. The use of an injectable polyvinyl alcohol gel according to any of the previous claims in the area of replacement and repair of intervertebral disks, in particular the nucleus.

## Revendications

1. Gel d'alcool polyvinylique injectable, **caractérisé en ce que**
a) ledit gel est formé, à des températures comprises entre 0 et 100 °C, à partir d'une solution ne comportant nécessairement que de l'alcool polyvinylique et de l'eau ; et
b) ledit gel comporte au moins un alcool polyvinylique de type PVA1 et au moins un alcool polyvinylique de type PVA2 ; et
c) le degré de polymérisation DPn de PVA1 est > 1000 ; et
d) le degré de polymérisation DPn de PVA2 est compris entre 50 et 1000; et
e) le degré d'hydrolyse de PVA1 et de PVA2, en % molaires, est > 98.

2. Gel d'alcool polyvinylique injectable, **caractérisé en ce que**
a) ledit gel est formé, à des températures comprises entre 0 et 100 °C, à partir d'une solution ne comportant nécessairement que de l'alcool polyvinylique et de l'eau et au moins un additif sous forme d'une charge ou d'un principe actif et/ou au moins un agent de gonflage et/ou au moins un autre polymère; et
b) ledit gel comporte au moins un alcool polyvinylique de type PVA1 et au moins un alcool polyvinylique de type PVA2 ; et
c) le degré de polymérisation DPn de PVA1 est > 1000 ; et
d) le degré de polymérisation DPn de PVA2 est compris entre 50 et 1000; et
e) le degré d'hydrolyse de PVA1 et de PVA2, en % molaires, est > 98.

3. Gel d'alcool polyvinylique injectable selon la revendication 2, **caractérisé en ce que** ledit autre polymère est choisi dans le groupe constitué de polycarbonates, polyacrylates et polyméthacrylates, de polyéthylèneglycols, de poly(oxyéthylènes), de polyvinylpyrrolidones, de polycaprolactones, d'hydrocolloïdes et de polysaccharides.

4. Gel d'alcool polyvinylique injectable selon l'une des revendications précédentes, **caractérisé en ce que** le degré d'hydrolyse de PVA2, le cas échéant également le degré d'hydrolyse de PVA1, en % molaires, est > 99,85, préférentiellement > 99,9, encore plus préférentiellement > plus 99,95 et, avec la plus grande préférence, > 99,98.

5. Gel d'alcool polyvinylique injectable selon l'une des revendications précédentes, **caractérisé en ce que** les plus petites macromolécules dans les répartitions de la masse moléculaire de PVA1 et PVA2 présentent un degré de polymérisation de > 40.

6. Gel d'alcool polyvinylique injectable selon l'une des revendications précédentes, **caractérisé en ce que**, aux alentours de la température corporelle, la formation du gel a lieu dans les conditions in situ.

7. Gel d'alcool polyvinylique injectable selon l'une des revendications précédentes, **caractérisé en ce que** ledit gel présente, lorsqu'il est soumis à une compression et/ou à un essai de traction à température ambiante, un module de Young compris entre 0,1 et 25 MPa.

8. Gel d'alcool polyvinylique injectable selon l'une des revendications précédentes, **caractérisé en ce que** ledit gel présente, après 1 h, au moins 10 % du module de Young et/ou de la résistance mécanique par rapport à l'état durci.

9. Gel d'alcool polyvinylique injectable selon l'une des revendications précédentes, **caractérisé en ce qu'**un film d'une épaisseur d'environ 0,5 mm qui a gonflé à température ambiante dans de l'eau distillée, présente au terme de 14 jours, exprimé en %, une perte de polymère < 7, de préférence < 3.

10. Utilisation d'un gel d'alcool polyvinylique injectable selon l'une des revendications précédentes à titre de remplacement dans des applications de gels et de gels d'alcool polyvinylique existants, notamment dans le domaine de la biomédecine, dans le domaine du génie tissulaire et du génie des structures de support, dans l'orthopédie et dans le domaine de la chirurgie plastique.

11. Utilisation d'un gel d'alcool polyvinylique injectable selon l'une des revendications précédentes dans le domaine du remplacement et de la réparation de disques intervertébraux, notamment du nucleus.
